# EUROPEAN PATENT SPECIFICATION

(11) **EP 1 478 638 B1**
(45) Date of publication and mention of the grant of the patent: **09.08.2006**
(21) Application number: 03706744.4
(22) Date of filing: 27.02.2003
(51) Int. Cl.: C07D 307/87, A61K 31/343, A61P 25/24

(54) **PURIFICATION OF CITALOPRAM**
REINIGUNG VON CITALOPRAM
PURIFICATION DE CITALOPRAME

(30) Priority: 27.02.2002 GB 0204682
(43) Date of publication of application: 24.11.2004
(73) Proprietor: Cipla Ltd., Mumbai 400 008 (IN)
(72) Inventor: HAMIED, Y., K., 2nd Floor, Windsor Villa, Mumbai 400026 (IN); KANKAN, Rajendra N., A-3/5, NBD Society, Mumbai 400 084, Maharashtra (IN); RAO, Dharmaraj R., Thane 400 601, Maharashtra (IN)
(74) Representative: Wain, Christopher Paul
(86) International application number: PCT/GB2003/000836
(87) International publication number: WO 2003/072564

(56) References cited:
- GB-A- 610 570
- GB-A- 2 361 697
- CHEMICAL ABSTRACTS, vol. 84, no. 21, 24 May 1976 (1976-05-24) Columbus, Ohio, US; abstract no. 147278, MURAMATSU M. ET AL: "Purification of human urokinase" XP002225343 & JP 75 160473 A (TOWA CHEMICAL IND. CO., LTD.) 25 December 1975 (1975-12-25)
- CHEMICAL ABSTRACTS, vol. 105, no. 10, 8 September 1986 (1986-09-08) Columbus, Ohio, US; abstract no. 85185, FUKUYAMA Y. ET AL: "Phtalides from ligusticum wallichi" XP002225344 & JP 61 007267 A (OTSUKA PHARM. CO., LTD.) 13 January 1986 (1986-01-13)

## Description

This invention relates to purification of citalopram.

Citalopram is a well known antidepressant drug whose systematic name is 1-[3-(dimethylamino)propyl]-1-(4-fluorophenyl)-1,3-dihydro-5-isobenzofurancarbonitrile. It is a selective centrally acting serotonin (S-hydroxytryptamine; 5-HT) reuptake inhibitor. It is marketed as the hydrobromide or hydrochloride salt. An important enantiomer is S-citalopram.

Citalopram was first described in GB-A-1526331 and, subsequently, a number of different processes have been described for its preparation. In many of these, the final step is to introduce the 5-cyano group but there have been problems in purifying the final product to remove intermediates and by-products. Among the purification processes used has been isolation of the free base as an oil (bp 175°C/0.03 mm Hg // 4 Pa) and subsequent thin film distillation followed by conversion to the desired salt. Another purification process involves conversion to a salt and recrystallisation thereof Neither of these techniques has been particularly satisfactory.

Recently, another purification procedure has been described in GB-B-2357762. Here, citalopram base is set free and precipitated in crystalline form, and after optional recrystallisation for purification, converted to the desired salt. This process is said to be particularly effective at removal of 5-substituted intermediate contaminants. However, a disadvantage of this process is that it requires repeated crystallisations to achieve high purity and this is undesirable.

We have now found another way of purifying citalopram which has a number of advantages over prior known processes.

In accordance with one aspect of the present invention, crude citalopram is purified by adsorption on a solid support and subsequent release therefrom. We have found that, in this way, very satisfactory purification can be achieved in a simple manner.

The process of the present invention is not chromatographic but rather uses solid support adsorbents which are not packed in a column. The adsorbents which are used in the present invention are preferably inert particulate materials which can be mixed with a liquid containing citalopram base to adsorb the base, and can then be filtered off or otherwise separated from the liquid. It is also possible to use other forms of adsorbent support.

The solid support adsorbent is one which will adsorb citalopram base. Those skilled in the art will be familiar with a large number of adsorbents, any of which can be routinely tested for suitability in the process of the invention. Among the preferred materials are, for example, particulate diatomaceous earth (eg celite), clay, alumina and silica. Other adsorbents include, for example, Zeolites of different types.

The crude citalopram which can be purified in accordance with the present invention may have been made in any way such as, for example, by any of the methods known in the art. The purification method of the present invention is particularly useful with crude citalopram made by a step including exchange of a 5-halo substituent by a 5-cyano substituent using, for example, sodium, potassium, cuprous or zinc cyanide, with or without catalysts or solvents.

In one example of the method of the invention, crude citalopram base is dissolved in an aqueous acidic solution. The acid can be an inorganic, or an organic, water-soluble acid. Preferred acids are, for example, hydrochloric acid, hydrobromic acid, acetic acid, sulphuric acid, oxalic acid and formic acid, but others can be used. The solid support adsorbent is then mixed with the acid solution containing crude citalopram, and the solution is then neutralized using a suitable base, to deposit the citalopram base in adsorbed state on the solid support. Preferably, the weight ratio of the crude citalopram to the solid support is from about 1:0.5 to about 1:20.

Upon adsorption of the citalopram base on the solid support and subsequent separation of the support from the liquid, any impurities remaining in the liquid are effectively removed.

In a preferred procedure according to the invention, before the adsorbed citalopram base is released (desorbed) from the solid support, the support is washed with solvents to leach out any adsorbed impurities without displacing the base, ie the base remains on the support at the end of the procedure. For this purpose, we prefer to use aliphatic hydrocarbon solvents such as hexane or heptane, optionally mixed with an aromatic solvent, eg an aromatic hydrocarbon such as toluene, or with lower (eg C_{1 to 4}) alcohols such as isopropanol or methanol, for example. The suitability (or otherwise) of any particular solvent or solvent mixture for leaching out impurities without significant removal of the base can be simply tested, as will be clear to those skilled in the art. Preferably, the solid support is treated with solvent(s) to remove the impurities at temperatures ranging from ambient to reflux, for periods of from a few minutes up to several hours, for example.

In one preferred embodiment, the solid support with adsorbed citalopram base thereon is heated with an organic solvent such as toluene, hexane is added and the mixture cooled. In this procedure, impurities are desorbed into the solvent and, hence, removed. Some of the base may desorb into the hot solvent but, on cooling, it is directly re-adsorbed on the support without crystallization so that the base is remaining on the support at the end of the procedure but free from the desorbed impurities.

In order to desorb the purified base from the solid support, we prefer to contact the support with polar solvents. There are many suitable such solvents but we prefer to use acetone, ethyl acetate, methanol, isopropanol or tetrahydrofuran. After treatment with a polar solvent, the solvent (containing the dissolved purified base) is separated from the spent solid support, such as by filtration.

The clear filtrate is a solution of the purified citalopram base. The base itself can be recovered from the solution if desired, such as in the form of an oil or as crystals. We prefer, however, to convert the base in solution directly to a desired salt, such as the hydrobromide, hydrochloride or oxalate. This can be effected by methods well known in the art such as by adding the appropriate acid to the solution of the base and then recovering the salt.

The purified base of the present invention is substantially free of impurities such as the 5-carboxamide impurity, 5-chloro impurity, 5-bromo impurity and the N-desmethyl impurity. Typically the level of each of these impurities in the purified citalopram is less than 0.1 %.

In order that the invention may be more fully understood, reference is made to the accompanying Examples.

### Example 1:

Crude citalopram base 10 g (HPLC purity 82%) is dissolved in 100 ml 5% aqueous acetic acid solution. To this is added 20 g of celite and the reaction mixture is neutralized with 5% sodium hydroxide to a pH of about 9. The reaction mixture is stirred for 3 hr at room temperature and filtered to give citalopram base supported on celite as a fine solid.

### Example 2:

Crude citalopram base 10 g (HPLC purity 87%) is dissolved in 100 ml 5% aqueous hydrobromic acid solution. To this is added 40 g of montmorillonite clay and the reaction mixture is neutralized with 5% ammonium hydroxide to a pH of about 9. The reaction mixture is stirred for 3 hr at room temperature and filtered to give citalopram base supported on clay as a fine solid.

### Example 3:

Crude citalopram base 10 g (HPLC purity 92%) is dissolved in 100 ml 5% aqueous hydrochloric acid solution. To this is added 30 g silica gel (60-120#) and the reaction mixture is neutralized with 5% sodium hydroxide to a pH of about 9. The reaction mixture is stirred for 3 hr at room temperature and filtered to give citalopram base supported on silica gel as a fine solid.

### Example 4:

The solid obtained from Example 1 was purified by suspending it in 100 ml hexane, stirring at 60°C for 2 hours, cooling to ambient and filtering. The solids were then treated again in the same way but using a 1:10 mixture of toluene:hexane, to obtain purified citalopram base (HPLC purity 99.3%) adsorbed on celite.

### Example 5:

The solid obtained from Example 2 was suspended in 100 ml hexane, stirred at 60°C for 2 hours, and then cooled to ambient and filtered. The process was repeated using a 1:5 mixture of toluene:heptane to obtain purified citalopram base (HPLC purity 99.6%) adsorbed on montmorillonite clay.

### Example 6:

The solid obtained from Example 3 was suspended in 100 ml hexane, stirred at 60°C for 2 hours, and then cooled to ambient and filtered. The process was repeated using a 1:15 mixture of isopropanol:hexane to obtain purified citalopram base (HPLC purity 99.5%) adsorbed on silica.

### Example 7:

30 g of fine solid obtained in Example 4 was stirred with ethyl acetate 120 ml at 50°C for 2 hr. The solution was filtered. To the clear filtrate was added aqueous hydrobromic acid and the pH adjusted to 3.5. The solution was cooled to 5°C and filtered to give pure citalopram hydrobromide (HPLC purity 99.6%).

In a similar manner, the solids obtained in Examples 5 and 6 were treated to release the purified citalopram base into solution and convert it to citalopram hydrobromide.

## Claims

1. A method of purifying citalopram base which comprises adsorbing the base on a solid support and subsequently releasing the purified base therefrom.

2. A method according to claim 1, wherein the solid support is in loose particulate form.

3. A method according to claim 1 or 2, wherein the support is diatomaceous earth, clay, silica or alumina.

4. A method according to claim 1, 2 or 3, wherein the weight ratio of citalopram base to solid support is from 1:0.5 to 1:20.

5. A method according to any of claims 1 to 4, wherein the solid support having said citalopram base adsorbed thereon is washed to leach out impurities.

6. A method according to claim 5, wherein the support is washed with an organic solvent comprising an aliphatic hydrocarbon, optionally in admixture with a lower alcohol or an aromatic solvent.

7. A method according to any of claims 1 to 6, wherein the citalopram base is desorbed from the support by washing with a polar solvent for the base.

8. A method according to claim 7, wherein the solvent is an aliphatic ketone, an ester, an open chain or cyclic ether, or a C₁ to C₄ alcohol, and particularly acetone, ethyl acetate, methanol, isopropanol, ethanol or tetrahydrofuran.

9. A method of making a pharmaceutically acceptable salt of citalopram which comprises directly converting a solution of citalopram base purified by the method of any of claims 1 to 8, to a pharmaceutically acceptable salt thereof

## Patentansprüche

1. Verfahren zur Reinigung der Base von Citalopram, das die Adsorption der Base an einen festen Träger und anschließend das Freisetzen der gereinigten Base davon umfasst.

2. Verfahren nach Anspruch 1, worin der feste Träger in loser partikulärer Form vorliegt.

3. Verfahren nach Anspruch 1 oder 2, worin der Träger Diatomeenerde, Ton, Siliziumdioxid oder Aluminiumoxid darstellt.

4. Verfahren nach Anspruch 1, 2 oder 3, worin das Gewichtsverhältnis der Base von Citalopram zu festem Träger von 1:0,5 bis 1:20 beträgt.

5. Verfahren nach einem der Ansprüche 1 bis 4, worin der feste Träger, der die genannte Base von Citalopram darauf adsorbiert aufweist, zum Herauslösen der Verunreinigungen gewaschen wird.

6. Verfahren nach Anspruch 5, worin der Träger mit einem organischen Lösungsmittel, umfassend einen aliphatischen Kohlenwasserstoff, optional in Beimischung mit einem niederen Alkohol oder einem aromatischen Lösungsmittel, gewaschen wird.

7. Verfahren nach einem der Ansprüche 1 bis 6, worin die Base von Citalopram aus dem Träger durch Waschen mit einem polaren Lösungsmittel für die Base desorbiert wird.

8. Verfahren nach Anspruch 7, worin das Lösungsmittel ein aliphatisches Keton, einen Ester, eine offene Kette oder einen cyclischen Ether oder einen C₁- bis C₄-Alkohol und insbesondere Aceton, Ethyl, Acetat, Methanol, Isopropanol, Ethanol oder Tetrahydrofuran darstellt.

9. Verfahren zur Herstellung eines pharmazeutisch verträglichen Salzes von Citalopram, das die direkte Umwandlung einer Lösung der Base von Citalopram, die durch das Verfahren nach einem der Ansprüche 1 bis 8 gereinigt wird, in ein pharmazeutisch verträgliches Salz davon umfasst.

## Revendications

1. Procédé de purification d'une base citalopram qui comprend l'étape consistant à adsorber la base sur un support solide et ensuite l'étape consistant à libérer la base purifiée de celui-ci.

2. Procédé selon la revendication 1, dans lequel le support solide est sous une forme particulaire libre.

3. Procédé selon la revendication 1 ou la revendication 2, dans lequel le support est de la terre de diatomées, de l'argile, de la silice ou de l'alumine.

4. Procédé selon la revendication 1, 2 ou 3, dans lequel le rapport en poids de la base citalopram sur le support solide est de 1 : 0,5 à 1 : 20.

5. Procédé selon l'une quelconque des revendications 1 à 4, dans lequel le support solide présentant ladite base citalopram adsorbée sur lui est lavé pour lixivier les impuretés.

6. Procédé selon la revendication 5, dans laquelle le support est lavé avec un solvant organique comprenant un hydrocarbure aliphatique, éventuellement en mélange avec un alcool inférieur ou un solvant aromatique.

7. Procédé selon l'une quelconque des revendications 1 à 6, dans lequel la base citalopram est désorbée du support par lavage avec un solvant polaire pour la base.

8. Procédé selon la revendication 7, dans lequel le solvant est une cétone aliphatique, un ester, un éther à chaîne ouverte ou cyclique, ou un alcool en C₁ à C₄, et particulièrement de l'acétone, de l'acétate d'éthyle, du méthanol, de l'isopropanol, de l'éthanol ou du tétrahydrofurane.

9. Procédé de fabrication d'un sel de citalopram pharmaceutiquement acceptable qui comprend l'étape consistant à convertir directement une solution de base citalopram purifiée par le procédé selon l'une quelconque des revendications 1 à 8, en un sel pharmaceutiquement acceptable de celle-ci.
